# EUROPEAN PATENT SPECIFICATION

(11) **EP 1 707 628 B1**
(45) Date of publication and mention of the grant of the patent: **11.11.2009**
(21) Application number: 05011839.7
(22) Date of filing: 01.06.2005
(51) Int. Cl.: C12N 15/10, A01K 67/027, C07K 16/00

(54) **Methods for producing recombinant polyclonal immunoglobulins**
Verfahren zur Produktion von rekombinanten polyklonalen Immunglobulinen
Procédé de production d'immunoglobulines polyclonales recombinantes

(30) Priority: 30.03.2005 US 92988; 31.03.2005 JP 2005104770
(43) Date of publication of application: 04.10.2006
(73) Proprietor: SEKISUI CHEMICAL CO., LTD., Osaka-shi, Osaka 530-8565 (JP); Suzuki, Kazuo, Isumi-gun, Chiba 299-4501 (JP)
(72) Inventor: Suzuki, Kazuo, Isumi-gun Chiba 299-4501 (JP); Furutani, Masahiro, Takatsuki-shi Osaka 596-1044 (JP); Yamamoto, Kenji, Tokyo 113-0024 (JP); Ohno, Naohito, Hachioji-shi Tokyo 192-0363 (JP); Aratani, Yasuaki, Yokohama-shi Kanagawa 233-0003 (JP); Togi, Akiko, Takatsuki-shi Osaka 569-0073 (JP); Takahashi, Kei, Yokohama-shi Kanagawa 241-0001 (JP)
(74) Representative: Schnappauf, Georg

(56) References cited:
- EP-A- 1 054 018
- EP-A- 1 516 929
- US-B1- 6 335 163
- DATABASE WPI Section Ch, Week 200313 Derwent Publications Ltd., London, GB; Class D16, AN 2003-132125 XP002365795 & JP 2002 262883 A (KAIYO BIOTECHNOLOGY KENKYUSHO KK) 17 September 2002 (2002-09-17)
- XIAO HONG ET AL: "Antineutrophil cytoplasmic autoantibodies specific for myeloperoxidase cause glomerulonephritis and vasculitis in mice" JOURNAL OF CLINICAL INVESTIGATION, vol. 110, no. 7, October 2002 (2002-10), pages 955-963, XP002366093 ISSN: 0021-9738
- ARATANI YASUAKI ET AL: "Severe impairment in early host defense against Candida albicans in mice deficient in myeloperoxidase" INFECTION AND IMMUNITY, vol. 67, no. 4, April 1999 (1999-04), pages 1828-1836, XP002365790 ISSN: 0019-9567
- FRANCOIS BANEYX; MIRNA MUJACIC: 'Recombinant protein folding and misfolding in Escherichia coli' NATURE BIOTECHNOLOGY vol. 22, November 2004, pages 1399 - 1408
- SAMBROOK J.; FRITSCH E.F.; MANIATIS T.: 'Molecular Cloning', 1989, COLD SPRING HARBOR LABORATORY PRESS * pages 1.3-1.5; table 1.1 *

## Description

### BACKGROUND OF THE INVENTION

### TECHNICAL FIELD

The present invention relates to a method for producing recombinant polyclonal scFvs, and more particularly, to a method for producing recombinant polyclonal scFvs which comprises culturing a plurality of types of transformants containing a plurality of types of immunoglobulin genes respectively, and obtaining polyclonal scFvs. The present invention provides constant supply of scFv preparations with minimum risk of infection

### DESCRIPTION OF RELATED ART

The immunoglobulin preparation, one of blood products purified from human blood plasma, has host-defense activity such as protection against bacteria and other invaders due to antigen-antibody reactions. Recently, immunoglobulin preparations have been adapted to treatments for diseases such as idiopathic thrombocytopenic purpura, agammaglobulinemia, acute-stage of Kawasaki disease and Guillain-Barre syndrome. High dosages of intravenous injection of immunoglobulin (IVIg) are major treatment for these diseases. The IVIg is paid attention as a treatment for intractable vasculitis. In addition, IVIg is expected to be applied to some diseases in the world. Recently, IVIg has been approved as a treatment for autoimmune disease Guillain-Barre syndrome. Furthermore, treatments with IVIg are being applied to new trial for collagen diseases and myasthenia. The IVIg has been effective in the treatments for Kawasaki disease for over 20 years. Recently, one shot of IVIg with 2g/Kg/day has been approved for the treatment of Kawasaki disease. The IVIg is useful for serious and intractable diseases. In addition, IVIg is a useful treatment in the viewpoint of lower side effects.

Several hypotheses for a mode of action of immunoglobulin treatments have been proposed. One is that a lot of antibodies contained in immunoglobulins show effective action against unknown antigens. The second is that anti-myeloperoxidase (MPO) antibodies contained in immunoglobulins show effective treatment due to action of wide-ranged polyclonal antibodies to MPO epitopes. Both hypotheses are same from the viewpoint that immunoglobulins contain polyclonal antibodies with wide varieties and this variety of antibodies contribute to the effective action for treatments. These two typical hypotheses seem to be most actual.

The immunoglobulin is a general term of antibodies and also proteins having association with antibody structures and functions. The immunoglobulin the antigen of which is determined, is called as an antibody against the determined antigen. Basic structure of immunoglobulin molecule is constructed with one molecule made of two light chains (L-chain) and two heavy chains (H-chain) and these chains are conjugated with disulfide bonds. H-chain is constructed with two regions; constant region (C-region) composed of three domains CH1, CH2 and CH3, and a variable region composed of VH region (V-region). L-chain is constructed with two regions; C-region composed of CL region and V-region composed of VL region. The amino acids sequences of the V-regions of antibodies synthesized in a body, are diversified to show specificity for various antigens.

Immunoglobulins have usually risks due to unknown pathogens such as virus because these are human blood products. Indeed, blood sources containing pathogenic virus have been served to blood products for therapies, causing social problems with the harmful effect of a medicine. In addition, decrease of blood source will be worried due to increase of use for newly application to therapies. Thus, in order to decrease of the infectious risks and define the mode of action of immunoglobulins for therapies, some synthetic immunoglobulins are demanded. For this purpose, establishment of the process composed of collection of plurality of antibody genes, synthesis of recombinant polyclonal immunoglobulins by recombinant DNA technology and purification of synthesized recombinant polyclonal immunoglobulins, is desired.

Production of recombinant antibodies by' using recombinant DNA techniques has been reported. For example, JP 5-304989A discloses production of a chimeric antibody in which the V-region is replaced with that of a mouse. JP 2000-14383A discloses production of a humanized antibody in which only CDR regions of the V-region are replaced with those of a mouse. In addition, chimeric antibodies and humanized antibodies are already launched into the market. Further, JP 2004-81199A discloses techniques producing soluble antibodies as fusion proteins with molecular chaperones expressed in host cells.

EP 1 516 929 A2 discloses methods for production of human antibody libraries including multiplex overlap-extension RT-PCR. However, these techniques for production of antibodies have been used for expression of monoclonal antibodies generated from a single gene. There is little available information of the highly-expression of recombinant polyclonal antibodies as established in the present invention.

As described above, the immunoglobulin is one of blood products and consisted of mixture of wide-varieties of immunoglobulins, that are polyclonal antibodies. It is believed that the mixture is effective for therapy. Thus, recombinant immunoglobulins produced by recombinant DNA techniques must be composed of polyclonal antibodies. However, it has never been established to produce recombinant polyclonal immunoglobulins with wide-varieties using recombinant DNA techniques.

The purpose of the present invention is to provide methods for producing recombinant polyclonal immunoglobulins using recombinant DNA techniques, which provides constant supply of immunoglobulin preparations with minimum risk of infection.

### SUMMARY OF THE INVENTION

One of aspects of the present invention is to provide a method for producing recombinant polyclonal immunoglobulins with inclusion body formation which comprises the following steps:
(1) preparing a plurality of types of recombinant vectors into which a plurality of types of genes is introduced, respectively, by contacting a mixture of a plurality of types of genes with vectors, wherein said genes have been obtained by isolating them from cDNAs derived from tissues or cells expressing immunoglobulins and wherein said genes encode a plurality of types of polypeptides containing a plurality of types of immunoglobulin variable regions, and preparing mixture of the recombinant vectors;
(2) preparing a plurality of types of transformants into which a plurality of types of recombinant vectors are introduced, respectively, by contacting said mixture of the recombinant vectors with Escherichia coli cells, and preparing a mixture of the transformants; and
(3) culturing said mixture of the transformants,
   and obtaining a mixture of polypeptides from the culture of the transformants, wherein said polypeptides contain a plurality of types of immunoglobulin variable regions respectively, and said polypeptides when expressed form inclusion bodies, wherein said polypeptides are scFv.

The method for producing recombinant polyclonal immunoglobulins as outlined above comprises a first step of isolating a plurality of types of genes, said genes encoding a plurality of types of polypeptides respectively, and said polypeptides containing a plurality of types of immunoglobulin variable regions (hereinafter referred to "V-regions") respectively, and preparing mixture of the genes. Thus, a plurality of types of genes corresponding to a variety of V-regions are isolated, and then mixture of the genes is prepared. In other words, a library of genes is prepared in the first step. Next, the method in the aspect comprises a second step of preparing a plurality of types of recombinant vectors into which a plurality of types of genes are introduced respectively by contacting said mixture of the genes with vectors, and preparing mixture of the recombinant vectors. In other words, a library of recombinant vectors is prepared in the second step. Next, the method in the aspect comprises a third step of preparing a plurality of types of transformants into which a plurality of types of recombinant vectors are introduced respectively by contacting said mixture of the recombinant vectors with host cells, and preparing mixture of the transformants. In other words, a library of transformants is prepared in the third step. Finally, the method in the aspect comprises a fourth step of culturing said mixture of the transformants, and obtaining mixture of polypeptides from the transformants culture, wherein said polypeptides contain a plurality of types of immunoglobulin variable regions respectively. The obtained polypeptides contain a plurality of types of immunoglobulin variable regions respectively, and thus mixture of the polypeptide constitutes "recombinant polyclonal immunoglobulins".

The term "immunoglobulins" of the present invention refers to artificial proteins/polypeptides containing V-regions such as scFv (single chain Fv) which are fusion proteins composed of VL and VH.

Herein, "the genes isolated from the cDNAs" include not only isolated genes continuously located on the cDNA but also fusion genes composed of two or more genes separately located on the cDNAs by combining the genes by an appropriate manner. Because genes encoding full-length or a part of the wild-type immunoglobulin, namely, full-length of native immunoglobulin such as H-chain (VH-CH1-CH2-CH3) and L-chain (CL-VL), and a part of the wild-type immunoglobulin such as CH1-VH, VH alone and VL alone are continuously located on the cDNAs, the genes can be isolated directly from the cDNAs with one step. On the other hand, genes encoding artificial antibody fragments lacking the complete structure of immunoglobulin such as scFv (VL-VH fragment) do not exist continuously. To isolate genes encoding scFv, genes encoding VL and genes encoding VH are combined and then isolated as continuous genes with an appropriate manner. The appropriate manners include overlap-extension-PCR method.

Recombinant polyclonal immunoglobulins produced by the method in the aspect contain a varieties in reactivity as well as native immunoglobulins derived from human blood plasma. Thus, the method for producing recombinant polyclonal immunoglobulins in the aspect provides immunoglobulin preparations having the same activity as that of immunoglobulin preparations from human blood plasma. The immunoglobulin preparations produced by the method in the aspect decrease the risk of infection with high safety.

scFv, which is called "single chain Fv antibody", is an artificial antibody consisting of a VL-VH fragment, a fusion protein composed of a VL region and a VH region. Since scFv is consisted of a VL region and a VH region, a minimum unit for an antibody to recognize an antigen, scFv is more concise in molecular size. scFv is known to be expressed and produced easily in E. coli. Thus, E. coli is used as host cells in the preferred aspect of the method for producing recombinant polyclonal immunoglobulins. Consequently, it is easier to produce recombinant polyclonal immunoglobulins by the method in the preferred aspect.

In the method for producing recombinant polyclonal immunoglobulins, the host cells are Escherichia coli. E. coli can be cultured easily at a large scale. Thus, this aspect provides mass production of recombinant polyclonal immunoglobulins, and constant supply of immunoglobulin preparations.

It is preferable that said tissues or cells are derived from mammals.

In the preferred aspect of the' method for producing recombinant polyclonal immunoglobulins, a plurality of types of genes from cDNAs derived from mammalian tissues or cells expressing immunoglobulins, said genes encoding a plurality of types of polypeptides respectively, and said polypeptides containing a plurality of types of immunoglobulin variable regions respectively, are isolated. And then, mixture of the genes is prepared. When the mammalian tissues or cells derived from human are used, completely human-type recombinant polyclonal immunoglobulins, useful for immunoglobulin preparations, can be produced. When the tissues or cells derived from other mammals except human such as mice are used, chimeric recombinant polyclonal immunoglobulins can be produced.

When producing foreign proteins using recombinant DNA techniques, the proteins must be obtained as normal proteins with correct three-dimensional structures. Folding factors are known as proteins assisting biosynthesized proteins to form correct three-dimensional structures.

It is preferable that said mammals are mice lacking myeloperoxidase genes.

In the preferred aspect of the method for producing recombinant polyclonal immunoglobulins, a plurality of types of genes from cDNAs derived from tissues or cells of mice lacking myeloperoxidase genes (MPO-deficient mice) expressing immunoglobulins, said genes encoding a plurality of types of polypeptides respectively, and said polypeptides containing a plurality of types of immunoglobulin variable regions respectively, are isolated. MPO-deficient mice injected with MPO produce a large amount of antibodies against MPO. Therefore, genes isolated from tissues or cells of MPO-deficient mice contain a large amount of genes encoding antibodies against MPO. Thus, the preferred aspect provides chimeric immunoglobulin preparations having effective and higher activity under a hypothesis that anti-MPO antibodies contained in immunoglobulins show effective and higher activity of immunoglobulin therapy using immunoglobulin preparations.

It is preferable that said folding factors are peptidyl prolyl cis-trans isomerases.

It is preferable that said peptidyl prolyl cis-trans isomerases are FK506 binding proteins.

It is preferable that said FK506 binding proteins are derived from archaea.

Peptidyl prolyl cis-trans isomerase (hereinafter referred to "PPIase"), one of the folding factors, is an enzyme having "PPIase activity" which catalyze cis-trans isomerization of peptide bonds in N-termini of proline residues of target proteins. However, PPIases derived from archaea (archaeal PPIases) are FK506 binding proteins and have also molecular chaperone activity in addition to PPIase activity. Herein, "molecular chaperone activity" is defined as activity to refold a denatured protein to an original normal form, or to repress a protein to form an irreversible aggregate. Therefore, archaeal PPIases can fold target proteins more correctly than other PPIases not having molecular chaperone activity. In these preferred aspects of the method for producing recombinant polyclonal immunoglobulins, the polypeptides containing V-regions are expressed as a part of fusion proteins composed of archaeal PPIases and the polypeptides. Consequently, the polypeptides containing V-regions can be obtained much more efficiently as proteins having correct three-dimensional structures. As a result, the polypeptides can be produced at higher yields to provide immunoglobulin preparations constantly.

Another aspect of the present invention is to provide a method for producing recombinant polyclonal immunoglobulins comprises the step of mixing the recombinant polyclonal immunoglobulins produced by the method of the above-specified aspect with polypeptides containing full-length or a part of CH1-CH2-CH3 regions.

The method for producing recombinant-polyclonal immunoglobulins in the aspect comprises mixing recombinant polyclonal immunoglobulins with polypeptides containing full-length or a part of C-regions. C-regions contribute to stability of immunoglobulins. Thus, the aspect of the method provides stable immunoglobulin preparations. The polypeptides containing full-length or a part of C-region can be obtained easily by using recombinant DNA techniques.

It is preferable that said polypeptides containing full-length or a part of CH1-CH2-CH3 regions are obtained from transformants culture and said transformants contain genes encoding said polypeptides.

In the preferred aspect of the method for producing recombinant polyclonal immunoglobulins, the polypeptides containing full-length or a part of CH1-CH2-CH3 regions are produced by culturing transformants. Consequently, the preferred aspect provides mass production of the polypeptides containing full-length or a part of CH1-CH2-CH3 regions, and constant supply of immunoglobulin preparations.

It is preferable that said polypeptides containing full-length or a part of CH1-CH2-CH3 regions are expressed as a part of fusion proteins composed of folding factors and said polypeptides containing full-length or a part of CH1-CH2-CH3 regions, and isolated from the fusion proteins.

In the preferred aspect of the method for producing recombinant polyclonal immunoglobulins, the polypeptides containing full-length or a part of CH1-CH2-CH3 regions are expressed as normal proteins more correctly. Consequently, the polypeptides can be produced at higher yields to provide immunoglobulin preparations more constantly.

It is preferable that said transformants are Escherichia coli.

In the preferred aspect of the method for producing recombinant polyclonal immunoglobulins, the polypeptides containing full-length or a part of CH1-CH2-CH3 regions can be produced at still higher yields to provide immunoglobulin preparations still more constantly.

It is preferable that said folding factors are peptidyl prolyl cis-trans isomerases.

It is preferable that said peptidyl prolyl cis-trans isomerases are FK506 binding proteins.

It is preferable that said FK506 binding proteins are derived from archaea.

In these preferred aspects of the method for producing recombinant polyclonal immunoglobulins, polypeptides containing full-length or a part of C-regions are expressed as normal proteins more correctly. Consequently, the polypeptides can be produced at higher yields to provide immunoglobulin preparations constantly.

### BRIEF DESCRIPTION OF THE DRAWINGS

Figure 1 is a schematic diagram showing constitution of expression vector pTmFKPI.
Figure 2 is a schematic diagram showing constitution of expression vector pTmFKPC123.

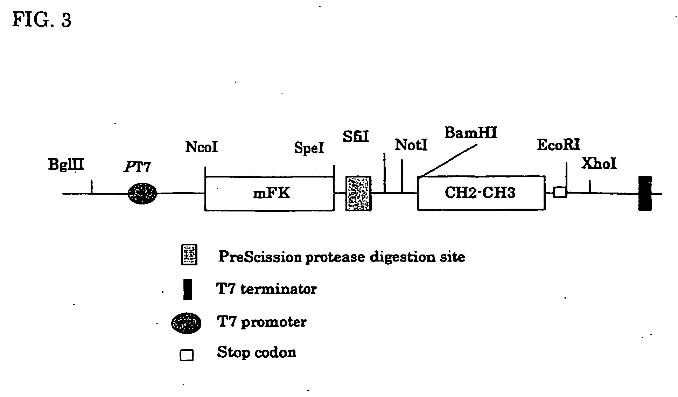

Figure 3 is a schematic diagram showing constitution of expression vector pTmFKPC23.
Figure 4 is a schematic diagram showing constitution of expression vector pTmFKPC.
Figure 5 is a schematic diagram showing molecular structures of polyclonal immunoglobulins composed of scFv-CH1-CH2-CH3 fragments produced in Example 3.
Figure 6 is a schematic diagram showing molecular structures of polyclonal immunoglobulins composed of scFv-CH2-CH3 fragments produced in Example 4.
Figure 7 is a schematic diagram showing molecular structures of scFv and CH1-CH2-CH3 fragnments which constitute polyclonal immunoglobulins produced in Example 5.
Figure 8 is a schematic diagram showing constitution of expression vector pT3DE.
Figure 9 is a photograph showing SDS-PAGE analysis of solubilization and refolding of recombinant polyclonal scFv antibodies.
Figure 10 is a graph showing suppression of vasculitis inducted with CAWS by injection with recombinant polyclonal immunoglobulin preparations.

### DESCRIPTION OF THE PREFERRED EMBODIMENTS

Preferred embodiments of the present invention are described in detail below.

One of the aspects of the method for producing recombinant polyclonal immunoglobulins of the present invention comprises four steps. These steps are explained below. The first step is, isolating a plurality of types of genes from cDNAs derived from tissues or cells expressing immunoglobulins, said genes encoding a plurality of types of polypeptides respectively, and said polypeptides containing a plurality of types of immunoglobulin variable regions respectively, and preparing mixture of the genes. In other words, a library of genes is prepared in the first step.

Tissues expressing immunoglobulins are spleen and lymphonode containing B cells and plasma cells producing antibodies. Spleen is better for isolation of genes. Hybridoma cells producing immunoglobulins are available for cell source for isolation of genes in addition to B cells and plasma cells. Several hybridoma cell lines must be used for obtaining the polyclonal immunoglobulins. For example, for several cell lines of hybridoma, hybridoma cell lines are prepared by several fused cell lines of B cells derived from MPO-deficient mouse with hybridoma cells. The polyclonal immunoglobulins are obtained from mixture of different hybridoma cell lines, which produce immunoglobulin having different epitopes recognizing MPO in each cell line. Using this procedure, the polyclonal immunoglobulins recognizing a specific antigen are obtained. In other words, recombinant polyclonal immunoglobulins based on any desired constructions (immunoglobulin molecules) can be produced by combining desired hybridomas. For this purpose, tissues or cells derived from mammals are preferred to the preparation. For example, complete human-type immunoglobulins are produced when tissues or cells derived from human are used. If tissues or cells are derived from other mammals, for example mouse, mouse-type polypeptides are produced, then chimeric immunoglobulins are produced by combination with human constant region of antibody.

The cDNAs derived from tissues or cells can be prepared by general methods For example, it is possible to synthesize first-strand cDNAs by reverse transcriptase reaction with mRNAs isolated from tissues or cells. Double-strands cDNAs are easily prepared by additional DNA synthesis. To isolate plural types of genes encoding variable regions of immunoglobulins from the obtained cDNAs, methods such as PCR in which target genes are amplified and isolated by a specific amplification, are generally available. In this case, the genes encoding the desired regions can be obtained by choosing the sequences of primers. For example, in the case of mouse genes, the diverse VL genes can be obtained by PCR with the mixture of oligonucleotides as shown by SEQ ID Nos. 1-18) as forward primers and the mixture of oligonucleotides as shown by SEQ ID Nos. 19-22 as reverse primers using cDNAs from a mouse spleen as templates. The VH genes also can be obtained by PCR with the mixture of oligonucleotides as shown by SEQ ID Nos. 23-41 as forward primers and the mixture of oligonucleotides as shown by SEQ ID Nos. 42-44 as reverse primers, in the same manner as VL genes. To obtain plural types of VL and VH genes, mixed-primers but not a single-primer, which are applicable to the plural variable regions, need to be used.

Furthermore, it is possible to link different two genes existing as discontinuous genes each other using the overlap-extension-PCR method. It is possible to make a scFv gene which is a non-native and artificial form of antibody genes by the method. In the case of mouse genes, it is possible to obtain plural types of scFv genes by PCR using plural types of VL and VH genes obtained as described above as the templates with oligonucleotides primers as shown by SEQ ID Nos. 45 and 46). Using primers containing restriction enzyme sites on the overlap-extension-PCR enables antibody genes to be easily inserted into expression vectors. The genes obtained as described above form libraries of plural types of genes encoding antibody variable regions.

The polypeptides containing immunoglobulin variable regions are scFv.

Since scFv consists of a VL region and a VH region, a minimum unit for an antibody to recognize an antigen, scFv is compact in molecular size. scFv is known to be expressed and produced easily in bacteria such as E. coli.

The CH1-VH region consists of the partial sequences of the H-chain of antibodies and contains the VH region, which is an essential unit for recognition of antibodies. The CL-VL region consists of whole sequence of the L-chain of antibodies, and contains the VL region, which is one of the essential units for recognition of antibodies. Polypeptides consisting of these regions are more concise in molecular size than H-chain of native antibodies.

The VH-region, a V-region of the H-chain of antibodies, is one of the essential units for recognition of antibodies. Polypeptides consisted of VH regions are more concise in molecular size than H-chain of native antibodies. Thus, the polypeptides in this embodiment express more easily. Consequently, it is easier to produce recombinant polyclonal immunoglobulins in this embodiment.

Fusion proteins composed of full-length or a part of CH1-CH2-CH3 regions and VH regions are, in other words, full-length or a part of heavy chain containing V-regions. The CH1-CH2-CH3 region (C-regions) of antibodies contributes to stability of antibodies. Therefore, the VH region contained in the fusion protein is more stable than the VH region alone.

The second step in the aspect is, preparing a plurality of types of recombinant vectors into which a plurality of types of genes are introduced respectively by contacting said mixture of the genes with vectors, and preparing mixture of the recombinant vectors. In other words, a library of recombinant vectors is prepared in the second step. Vectors applicable to kinds of host cells should be chosen in this embodiment. For example, as vectors applicable to E. coli hosts, any of vectors which contain such as an appropriate promoter, the SD sequence and a terminator, is available. As types of promoters, lac, tac, trc, trp, lambda-pL, T7, T3, etc are available. Further, high-copy plasmids are desirable from the viewpoints of productivity. For example, ColEI, pBR, pACYC derivatives are preferable. Such vectors are put on the market and thus can be easily obtained. To use vectors containing T7 promoters, E. coli DE3 strain need to be used in general. However, E. coli other than DE3 strains can be used by a method to add the CE6-phage to cultures in the logarithmic-growth-stage of the E. coli hosts.

As a method to introduce genes to vectors, the reaction by ligases is representative. For example, vectors are digested with restriction enzymes and then the target genes are introduced into the vectors by a ligase. As the types of ligases, T4 DNA ligase is representative. To introduce the PCR products to vectors, the TA-cloning method without restriction enzymes is available. Furthermore, a method by homologous-recombination, in which ligase-reactions are not required, is also available. For example, the target genes can be introduced into vectors by homologous-recombination using cre-recombinase (Clontech Co.) or lambda-integrase (Invitrogen Corp.) without ligases.

The third step in the aspect is, preparing a plurality of types of transformants into which a plurality of types of recombinant vectors are introduced respectively by contacting said mixture of the recombinant vectors with host cells, and preparing mixture of the transformants. In other words, a library of transformants is prepared in the third step. Particularly, many host-vector systems using E. coli as host cells have been established and these systems are suitable for this step. As a method to introduce vectors into host cells, individual methods are applicable according to kinds of host cells. For E. coli, methods such as calcium chloride treatment or electroporation are available.

The fourth step in the aspect is, culturing said mixture of the transformants, and obtaining mixture of polypeptides from the transformants culture, wherein said polypeptides contain a plurality of types of immunoglobulin variable regions respectively. Procedures for culturing transformants are not restricted specifically. The procedures can comprise, for example, inoculating mixture of the transformants to an appropriate medium and then culturing them with aeration and agitation. When recombinant vectors introduced into the transformants have antibiotic-resistant genes, the antibiotic corresponding to the genes can be added to the medium to avoid destruction of the recombinant vectors. When recombinant vectors introduced into transformants have inducible promoters, the inducers suitable for the promoter can be added to the medium to induce and regulate expression of immunoglobulin genes. When recombinant vectors introduced into transformants have thermo-sensitive promoters, increase in temperature for culturing can induce and regulate expression of immunoglobulin genes.

Mixture of polypeptides containing a plurality of types of immunoglobulin variable regions respectively can be obtained, for example, by collecting the cultured transformant cells by centrifugation, obtaining extract of the cells by homogeniziation, and obtaining the polypeptides from the extract. The mixture of polypeptides obtained by such manners constitutes "recombinant polyclonal immunoglobulins". Common procedures such as mechanical extraction can be used in cell homogenization. For example, homogenization using homogenizer, change of pressure and ultra-sonication can be used. Further, treatments with hypotonic and enzymatic conditions are also available.

Archaea from which FKBP type PPIases are obtained include Thermococcus sp., Methanococcus sp., Methanosarcina sp., Halobacterium sp., Archaeoglobus sp., Aeropurum sp., Purobaculum sp., Sulfolobus sp., Ferroplasma sp., Thermoplasma sp., Methanopyrus sp., Methanothermobacter sp. and Pyrococcus sp.

The method for producing recombinant polyclonal immunoglobulins in the aspect also comprises producing new polyclonal immunoglobulins by mixing polyclonal immunoglobulins produced by the four steps described above with other polyclonal immunoglobulins produced by the same. The embodiment provides polyclonal immunoglobulins consisting of two or more kinds of polypeptides. In the embodiment, mixing procedures is carried out preferably under oxidizing conditions for binding with disulfide bonds between these polypeptides. For example, as a CH1 region contains a cysteine residue and a CL region contains a cysteine residue, a polypeptide containing a CH1 region and a polypeptide containing a CL region can be bound by a disulfide bond. Thus, to mix a polypeptide composed of a CH1-VH region with a polypeptide composed of a CL-VL region provides an Fab fragment. Further, for example, as a CH2 region contain two cysteine residues, a polypeptide containing a CH1 region, a polypeptides containing a CL region, and a polypeptide containing a CH2 region can be bound by two disulfide bonds. Still further, a polypeptide containing CH2 region and another polypeptide containing CH2 region can be bound by a disulfide bond. Thus, to mix a polypeptide composed of a CL-VL region with a fusion protein composed of a VH region and a CH1-CH2-CH3 region provides a native immunoglobulin or an F(ab')₂ fragment.

Another aspect of the method for producing recombinant polyclonal immunoglobulins comprises mixing recombinant polyclonal immunoglobulins produced by the method of the first aspect above-described with polypeptides containing full-length or a part of C-regions. C-regions contribute to stability of immunoglobulins. Thus, the method of this aspect provides stable immunoglobulin preparations. The polypeptides containing full-length or a part of C-region can be obtained easily by using recombinant DNA techniques.

Like the first aspect described above, polypeptides containing V-region and/or polypeptides containing full-length or a part of C-regions are correctly folded by the effect of folding factors such as molecular chaperones in the embodiment. For example, polypeptides correctly folded can be isolated from fusion proteins composed of the polypeptides and folding factors. Archaeal FKBP type PPIases are preferably used as the folding factors.

The recombinant polyclonal immunoglobulins prepared from the transformants, which are highly purified with chromatographies etc., are provided as a drug substance for immunoglobulin preparations. General procedures for protein purification such as chromatography, salting out and ultrafiltration are applied to purification of the recombinant polyclonal immunoglobulins. Purity and other phisico-chemical characters of the recombinant polyclonal immunoglobulins obtained are analyzed by electrophoresis etc.

When expressed polypeptides containing antibody fragments form inclusion bodies, the polypeptides can be refolded by solubilizing the inclusion bodies with denaturants such as detergents.

Additives such as stabilizers and excipients can be added to the purified recombinant polyclonal immunoglobulin, a drug substance, to produce immunoglobulin preparations. Formulations for the immunoglobulin preparations include lyophilized formulations for injection, liquid formulations for injection, and liquid formulations for infusion. General additives used for protein formulations such as albumin, saccharides, polyoles and buffers can be used for the immunoglobulin preparations.

Recombinant polyclonal immunoglobulins which can be produced by the method of the present invention are fusion proteins containing V-regions namely scFv.

### EXAMPLES

Examples of the present invention are described in detail below, though the invention is not limited thereto.

### (EXAMPLE 1)

### Preparation of mouse antibody genes and construction of expression vectors.

### 1. Preparation of mouse single-chain-Fv (scFv) genes (I).

The mRNAs were extracted from spleens of MPO (Myeloperoxidase)-knockout mice (Y. Aratani et al. Infection and Immunity 67: 1828-36, 1999) with a RNA Extraction Kit (Amersham Biosciences Co.) and a mRNA Purification Kit (Amersham Bioscioces Co.). The cDNAs were synthesized using an oligo-dT primer with a First-Strand cDNA Synthesis Kit (Amersham Biosciences Co.). Mouse antibody VL genes were amplified by hot-start-PCR in the solutions (100 micro liters) containing 100 micro grams of the templates cDNAs, 100 pmole of the forward primers as shown by SEQ ID Nos. 1-18) and 100 pmole of the reverse primers as shown by SEQ ID Nos. 19-22 with thirty-cycles of the 4-steps; at 94 °C for 30 seconds, 63 °C for 30 seconds, 56 °C for 30 seconds, and 72 °C for 60 seconds using a TaKaRa Ex Taq Hot Start Version (Takara Bio Inc., Japan). Ten PCRs described above (100 micro liters) were conducted. The mouse VH genes were amplified in the same manner with the VL genes amplification using the forward primers as shown by SEQ ID Nos. 23-41 and the reverse primers as shown by SEQ ID Nos. 42-44. The amplified VL and VH genes were isolated by a 2.0% agarose-gel electrophoresis.

The scFv genes were synthesized by overlap-extension-PCRs (10 tubes of a 100 micro L reaction mixture) using 100 micro grams of the VL and VH genes as templates and the oligo-nucleotides as shown by SEQ ID Nos. 45 and 46 as the primers with a TaKaRa Ex Taq Hot Start Version (Takara Bio Inc., Japan). The PCRs were conducted with 7-cycles of the 4-steps; at 94 °C for 30 seconds, 63 °C for 30 seconds, 58 °C for 30 seconds, and 72 °C for 90 seconds, and with 23-cycles of the 3-steps; at 94 °C for 30 seconds, 63 °C for 30 seconds, and 72 °C for 90 seconds. The synthesized scFv genes were isolated by a 1.5% agarose gel electrophoresis. Ten micro-grams of scFv gene were obtained from individual mice. The SfiI I and Not I sites were introduced at the 5'- and 3'-ends of the scFv genes, respectively.

### 2. Construction of an expression vector pTmFKPI.

The genomic DNA was extracted from an archaeon, Methonosarcina acetivorans C2A (DSM2834). The FKBP (FK506 binding protein)-type peptidyl prolyl cis-trans isomerase (hereinafter referred to "mFK") gene (SEQ ID No. 49; GenBank number, NC003552) was amplified by PCR using the extracted genomic DNA as a template and the oligo-nucleotides as shown by SEQ ID Nos. 47 and 48 as primers. The amplified gene has Nco I and Spe I sites at the 5' and 3'-terminal ends of it, respectively. The improved pET21d was made by insertion of the synthetic DNA as shown by SEQ ID No. 50 into the Nco I-Xho I site of an expression vector pET21d (Novagen Co.). The inserted synthetic DNA as shown by SEQ ID No. 50 has a NcoI site (5'-CCATGG-3') at the 5'-end, a Xho I site (5'-CTCGAG) at the 3'-end, a Spe I site (5'-ACTAGT-3' corresponding to base numbers 11-16), a Not I site (5'-GCGGCCGC-3' corresponding to base numbers 61-68), two nucleotides sequences (5'-CTGGAAGTTCTGTTCCAGGGGCCG-3'corresponding to base numbers 18-41 and 69-91) encoding PreScission protease (Amersham Biosciences Co.) digestion sites, a nucleotides sequence (5'-CATCGCCATCGCCATCGC-3' corresponding to base numbers 96-113) encoding six histidine residues, and tandem stop codons (5'-TAATAG-3' corresponding to base numbers 114-119).

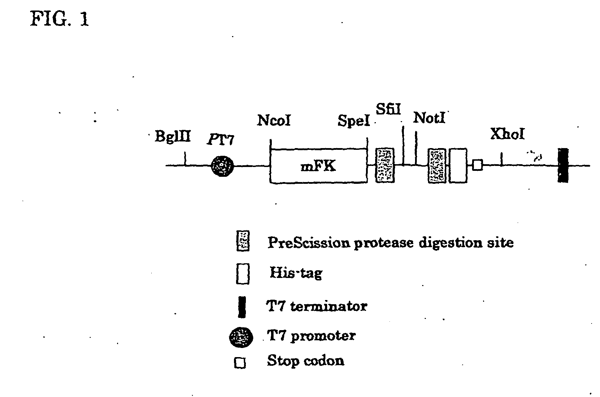

The amplified mFK gene digested with Nco I-Spe I was introduced into the Nco I-Spe I site of the improved pET21d and an expression vector pTmFKPI was constructed. The construction of pTmFKPI is shown in FIG. 1. This expression vector has T7- promoter and -terminator system. A fusion protein comprising mFK and a passenger protein is synthesized by insertion of a passenger gene into the Sfi I-Not I site of pTmFKPI. The fusion proteins are synthesized in the soluble fraction of E. coli cytoplasm because mFK has chaperone-function. A synthesized fusion protein has a Histidine-tag (His-tag) and is purified by a metal-chelating chromatography. Furthermore, a passenger protein is liberated from the His-tagged fusion protein by digestion with PreScission protease.

### 3. Construction of an expression vector pTmFKPC123.

The gene encoding a human antibody heavy chain constant region as shown by SEQ ID No. 53 and containing a stop codon was amplified by PCR from a human spleen cDNA library (Code No. 9509; Takara Bio Inc., Japan) using the oligo-nucleotides as shown by SEQ ID Nos. 51 and 52 as the primers: The amplified gene was cloned into a pT7 Blue-T vector (Novagen Co.) and the sequence of the inserted DNA fragment was confirmed by a Thermo Sequenase Dye Terminator Cycle Sequencing Kit (Amersham Biosciences Co.). The BamH I-EcoR I site was generated in pTmFKPI by insertion of a synthetic DNA as shown by SEQ ID No. 54 into the Not I-Xho I site of pTmFKPI. The synthetic DNA as shown by SEQ ID No. 54 has a Nco I-site (5'-CCATGG-3') at the 5'-end, a Xho I-site (5'-CTCGAG-3') at the 3'-end, a BamH I-site ( 5'-GGATCC-3' corresponding to base numbers 9-14), and an EcoR I-site. (5'-GGATCC-3' corresponding to base numbers 21-26).

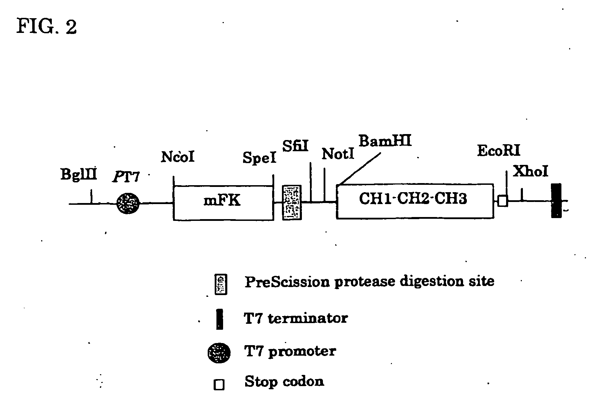

The cloned human antibody heavy chain constant region (CH1-CH2:CH3) gene containing a stop codon was introduced into the BamH I-EcoR I site of the above expression vector and an expression vector pTmFKPC123 (FIG. 2) was constructed. This expression vector has T7-promoter and -terminator system from pET21d (Novagen Co.). A fusion protein comprising mFK, a passenger protein, and CH1-CH2-CH3 is synthesized from pTmFKPC123 by insertion of a passenger protein gene into the SfiI-NotI site. The fusion protein is synthesized in the soluble fraction of E. coli cytoplasm because mFK has chaperone function. A fusion protein comprising a passenger protein and CH1-CH2-CH3 is easily liberated from mFK by digestion with PreScission protease.

### 4. Construction of an expression vector pTmFKPC23

The gene encoding human antibody Fc domain (CH2-CH3; SEQ ID No. 57) and stop codons was amplified by PCR from CH1-CH2-CH3 gene obtained in section 2 using oligo-nucleotides as shown by SEQ ID Nos. 55 and 56 as the primers. The amplified CH2-CH3 gene digested with BamH I-EcoR I was introduced into the BamH I-EcoR I site of pTmFKPC123 and an expression vector pTmFKPC23 was constructed. This expression vector has T7-promoter and -terminator system from pET21d (Novagen Co.). A fusion protein comprising mFK, a passenger protein, and CH2-CH3 is synthesized from pTmFKPC23 by insertion of a passenger protein gene into the Sfi I-Not I site. The fusion protein is highly synthesized in the soluble fraction of E. coli cytoplasm because mFK has chaperon function. The fusion protein comprising a passenger protein and CH2-CH3 is easily liberated from mFK by digestion with PreScission protease.

### 5. Construction of an expression vector pTmFKPHC

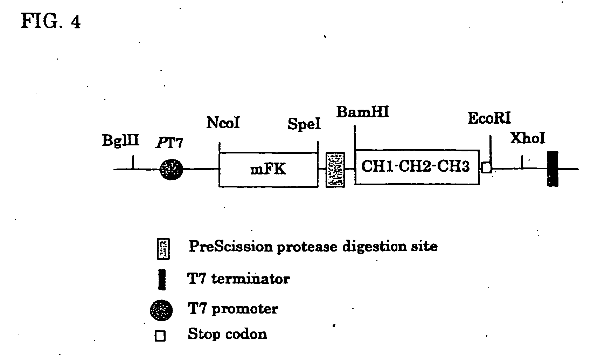

An expression vector pTmFKPHC (FIG. 4) was constructed by insertion of a synthetic DNA as shown by SEQ ID No. 58 into the Spe I-BamH I site of pTmFKPC123. The inserted synthetic DNA as shown by SEQ ID No. 58 has a Spe I site (5'-ACTAGT-3') at the 5'-end, a BamH I site (5'-GGATCC-3') at the 3'-end, and a sequence (5'-CTGGAAGTTCTGTTCCAGGGGCCG-3' corresponding to base numbers 7-30) coding the PreScission protease digestion site. The pTmFKPHC has T7-promoter and -terminator system from pET21d (Novagen Co.). A fusion protein comprising mFK and CH1-CH2-CH3 is synthesized from pTmFKPHC. The fusion protein is highly expressed in the soluble fraction of E. coli cytoplasm because mFK has chaperone function. The CH1-CH2-CH3 protein is liberated from mFK by digestion with PreScission protease.

### (Comparative EXAMPLE 2)

### Production of recombinant polyclonal single chain Fv (scFv) antibodies using a PPIase fusion system.

The amplified scFv genes from a MPO-knockout mouse obtained in Example 1 was digested with Sfi I-Not I and 200 ng of the digested scFv genes were introduced into the Sfi I-Not I site of pTmFKPI digested with Sfi I-Not I and treated with bacterial alkaline phosphates by T4 DNA ligase. E. coli JM109 (DE3) (Clontech Co.) (300 micro liters) was transformed with the ligase-reaction-mixture containing 500 ng DNA by electroporation (2.5 kv, 25 micro F, 200 Ω, 4.68 msec.). The SOC medium was added to the transformed cells and then incubated at 37 °C for 3 hours. The cell suspension (5 mL) was inoculated in 800 mL of 2 X Y.T. medium containing carbenicillin (100 micro grams/mL) and culture was conducted at 35 °C for 24 hours at the rotation of 140 rpm. After the culture, cells were harvested by centrifugation and disrupted by sonication in 50 mL of PBS. The supernatant was obtained by centrifugation.

The obtained supernatant was applied to a Hi-Trap Ni-chelating column (Amersham Biosciences Co.) equilibrated with 50 mM Tris-HCl buffer (pH 7.5) containing 0.5 M NaCl and then the fraction of mFK-scFv fusion protein was obtained by a linear gradient with 1 to 300 mM imidazole. The obtained fraction was dialyzed against 50 mM Tris-HCl (pH 7.0) containing 1 mM EDTA, 1 mM DTT and 150 mM NaCl overnight. In order to liberate the polyclonal scFv antibodies from the fusion proteins, PreScission protease (20 micro liters) was added to the dialysate and then incubated at 5 °C for 16 hours. After the digestion by PreScission protease, the solution was dialyzed against PBS containing 1 mM EDTA, 1 mM oxidized-form glutathione (GSSG) and 0.3 mM reduced-form glutathione (GSH). The dialysate was applied to a Protein L column (Pierce Co.) and polyclonal scFv antibodies were eluted by 100 mM Glycine-HCl buffer (pH 2.8). The scFv fraction was dialyzed against PBS containing 1 mM EDTA and stored at 4 °C.

The purified polyclonal scFv antibodies were obtained at the yield of approximately 20 mg/L culture. On the other hand, 100 micro liters of the 1000-fold diluted JM109 (DE3) cells transformed with ligase-reaction mixture and suspended in SOC medium was spread on LB-agar plates and the grown colony number was counted. The number of clones in the culture for scFv production, which corresponds to diversity of the synthesized polyclonal scFv antibodies, was estimated to be 2.7 X 10 ⁶.

### (Comparative EXAMPLE 3)

### Production of recombinant polyclonal scFv-CH1-CH2-CH3 antibodies by a PPIase fusion system.

The amplified scFv genes (200 ng) obtained in Example 1 was introduced into pTmFKPC123 (1 micro gram) shown in FIG. 2 by T4 DNA ligase. The JM109 (DE3) (300 micro liters) was transformed with the ligase-reaction-mixture containing 500 ng DNA by electroporation in the same condition as Example 2. After transformation, cells were suspended in 10 mL of SOC medium and incubated at 37 °C for 3 hours. The cell suspension (5 mL) was inoculated in 800 mL of 2 X Y.T. medium and culture was conducted at the rotation of 140 rpm at 35 °C for 24 hours. After the culture, cells were harvested by centrifugation and disrupted by sonication in 50 mL of PBS. The supernatant was obtained by centrifugation.

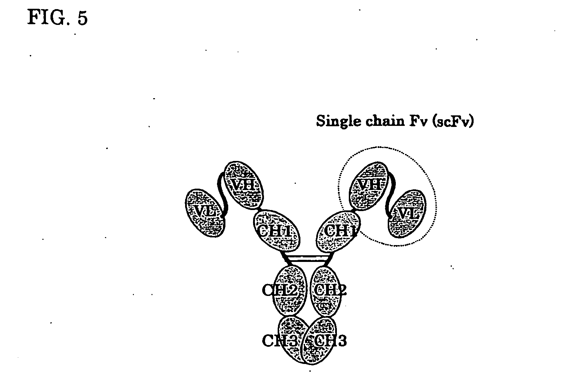

The obtained supernatant was applied to a Protein A column (Amersham Biosciences Co.) and the column was washed with 50 mM Tris-HCl (pH 7.5) containing 1mM EDTA. The synthetic polyclonal antibodies bound were eluted by 0.1 M Glycine-HCl buffer (pH 2.8). The obtained antibody fraction was dialyzed against 50 mM Tris-HCl buffer (pH 7.0) containing 1mM EDTA, 1 mM DTT, and 150 mM NaCl. To liberate polyclonal scFv-hFC123 (hFC123 shows CH1-CH2-CH3) antibodies from mFK, 20 micro liters of PreScission protease was added to the dialysate. The digestion reaction was conducted at 5 °C for 16 hours. After digestion, the reaction mixture was dialyzed against PBS containing 1 mM EDTA, 1 mM GSSG and 0.3 mM GSH. The dialysate was applied to a Protein A column and polyclonal scFv-hFC123 antibodies were eluted by 0.1 M Glycine-HC1 buffer (pH 2.8). The eluted polyclonal scFv-hFC123 antibodies were dialyzed against PBS containing 1 mM EDTA and stored at 4 °C. The structure of polyclonal scFv-hFC123 antibodies is shown in FIG. 5. This protein is a synthetic human/mouse chimeric antibody besides with the constant region of the light chain of immunoglobulin.

The purified polyclonal scFv-hFC123 antibodies were obtained at the yield of 5 mg/L culture. On the other hand, 100 micro liters of the 1000-fold diluted JM109 (DE3) cells transformed with the ligase-reaction mixture and suspended in SOC medium was spread on LB-agar plates containing ampicillin (100 micro grams/mL) and the number of grown colonies was counted. The number of clones in the culture for scFv-hFC123 production, which corresponds to diversity of the synthesized polyclonal scFv-hFC123 antibodies, was estimated to be 1.1 X 10 ⁶.

### (Comparative EXAMPLE 4)

### Production of recombinant polyclonal scFv-CH2-CH3 antibodies by a PPIase fusion system.

The amplified scFv genes (200 ng) obtained in Example 1 was introduced into the pTmFKPC23 (1 micro gram) shown in FIG. 2 by T4 DNA ligase. The JM109 (DE3) (300 micro liters) was transformed with the ligase-reaction-mixture containing 500 ng DNA by electroporation in the same condition as Example 2. After transformation, cells were suspended in 10 mL of SOC medium and incubated at 37 °C for 3 hours. The cell suspension (5 mL) was inoculated in 800 mL of 2 X Y.T. medium and culture was conducted at the rotation of 140 rpm. at 35 °C for 24 hours. After the culture, cells were harvested by centrifugation and disrupted by sonication in 50 mL of PBS. The supernatant was obtained by centrifugation.

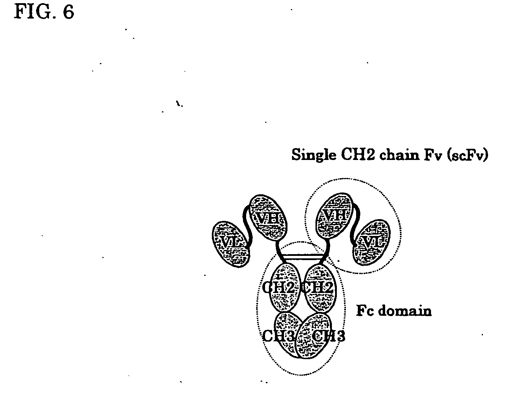

The obtained supernatant was applied to a Protein A column (Armeraham Biosciences Co.) and the column was washed with 50 mM Tris-HCl (pH 7.5) containing 1 mM EDTA. The synthetic polyclonal antibodies were eluted by 0.1 M Glycine-HCl buffer (pH 2.8). The obtained antibody fraction was dialyzed against 50 mM Tris-HCl buffer (pH 7.0) containing 1 mM EDTA, 1 mM DTT, and 150 mM Nacl. To liberate polyclonal scFv-hFC23 (hFC23 shows CH2-CH3) antibodies from mFK, 20 micro liters of PreScission protease was added to the dialysate. The digestion reaction was conducted at 5 °C for 16 hours. After digestion, the reaction mixture was dialyzed against PBS containing 1 mM EDTA, 1 mM GSSG, and 0.3 mM GSH. The dialysate was applied to a Protein A column and polyclonal scFv-hFC23 antibodies were eluted by 0.1 M Glycine-HCl buffer (pH 2.8). The eluted polyclonal scFv-hFC23 antibodies were dialyzed against PBS containing 1 mM EDTA and stored at 4 °C. The structure of polyclonal scFv-hFC23 antibodies is shown in FIG. 6. This protein is a synthetic human/mouse chimeric antibody comprising Fv (VL and VH) and Fc (CH2-CH3) domains.

The purified polyclonal scFv-hFC23 antibodies were obtained at the yield of 8 mg/L culture. On the other hand, 100. micro liters of the 1000-fold diluted JM109 (DE3) cells transformed with ligase-reaction mixture and suspended in SOC medium was spread on LB-agar plates containing ampicillin (100 micro grams/mL) and the number of grown colonies was counted. The number of clones in the culture for scFv-hFC23 production, which corresponds to diversity of the synthesized polyclonal scFv-hFC23 antibodies, was estimated to be 1.6 X 10 ⁶.

### (Comparative EXAMPLE 5)

### Production of the recombinant human antibody heavy chain constant region (CH1-CH2-CH3) by a PPIase fusion system.

E. coli BL21 star (DE3) (Invitrogen Co.) was transformed with pTmFKPHC constructed in Example 1 (FIG. 4). Ten clones of grown on a LB plate containing ampicillin (100 mg/mL) were inoculated in 800 mL of 2 X Y.T. medium. The culture was conducted at the rotation of 140 rpm at 35 °C for 20 hours. After the culture, cells were harvested by centrifugation and disrupted by sonication in 50 mL of PBS. Supernatant was obtained by centrifugation. The supernatant was applied to a Protein A column and the fusion protein was eluted by 0.1 M Glycirie-HCl buffer (pH 2.8). The eluted protein was dialyzed against 50 mM Tris-HCl buffer (pH 7.0) containing 1 mM EDTA, 1 mM DTT, and 150 mM NaCl. PreScission protease (20 micro liters) was added to the dialysate and the digestion was conducted at 5 °C for 15 hours. After digestion, the reaction mixture was dialyzed against PBS containing 1 mM EDTA, 1 mM GSSG, 0.3 mM GSH, and 150 mM NaCl. The dialysate was applied to a Protein A column again and the recombinant hFC123 was eluted by 0.1 M Glycine-HCl buffer (pH 2.8). The eluted protein was dialyzed against 50 mM Tris-HCl (pH 8.0) and applied to a Super Q-5PW column (TOSOH Co., Japan) equilibrated with 50 mM Tris-HCl (pH 8.0). The hFC123 was purified by a linear gradient with 0-500 mM NaCl. The purified recombinant hFC123 was dialyzed against PBS containing 1 mM EDTA. The purified recombinant hFC123 was obtained at the yield of 20 mg/L culture. The dimer formation of hFC123 was confirmed by SDS-PAGE under unreduced condition and gel filtration with a TSK gel G3000 SW-XL column (TOSOH Co., Japan). This result indicates that purified hFC123 was correctly folded and has native-like form of the immunoglobulin heavy chain constant region.

### (Comparative EXAMPLE 6)

### Preparation of the recombinant immunoglobulin containing recombinant polyclonal scFv antibodies and the Fc domain.

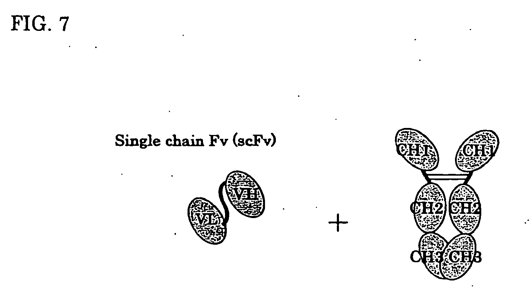

Equal molars of the recombinant polyclonal scFv antibodies obtained in Example 2 and recombinant hFC123 obtained in Example 5 were mixed. Figure 7 shows this recombinant immunoglobulin containing Fv and Fc domains. This type of recombinant immunoglobulin exhibits the similar functions with those of native immunoglobulin, recombinant scFv-hFC123 (Example 3), and scFv-hFC23 (Example 4). Furthermore, the molecular sizes of the components of this example are smaller than those of scFv-hFC23, and acFv-hFC123. This indicates that the components in this example have advantages in production yield with E. coli system.

### (EXAMPLE 7).

### Preparation of mouse single-chain-Fv (scFv) genes (II).

The mouse antibody Fv (VL and VH) cDNAs were synthesized using antibody specific primers as shown by SEQ ID Nos. 59-61 with a SuperScript III First Strand Synthesis System (Invitrogen Co.). The mouse spleen RNA prepared in Example 1 was used as a template for cDNA synthesis. The VL genes were amplified by hot-start-PCR in the solutions (100 micro liters) containing 100 micro grams of the templates cDNAs, 100 pmole of the forward primers as shown by SEQ ID Nos. 62-79 and 100 pmole of the reverse primers as shown by SEQ ID Nos. 19-22 with 30 cycles of the 3-steps; at 94 °C for 15 seconds, 56 °C for 30 seconds, and 72 °C for 90 seconds, followed by 72 °C for 10 minutes using a TaKaRa Ex Taq Hot Start Version (Takara Bio Inc., Japan). Ten PCRs reactions described above (100 micro liters) were conducted. The VH genes were amplified in the same manner as the VL genes amplification using the forward primers as shown by SEQ ID Nos. 23-41 and the reverse primers as shown by SEQ ID Nos. 42-44. The amplified VL and VH genes were isolated by a 2.0% agarose-gel electrophoresis.

The scFv genes were synthesized by overlap-extension-PCRs (10 tubes of a 100 micro liters reaction mixture) using 100 micro grams of the VL and VH genes as templates and the oligo-nucleotides as shown by SEQ ID Nos. 80 and 81 as the primers with a TaKaRa Ex Taq Hot Start Version (Takara Bio Inc., Japan). The PCRs were conducted with 23 cycles of the 3-steps; at 94 °C for 15 seconds, 56 °C for 30 seconds and 72 °C for 2 minutes, followed by 72 °C for 10 minutes. The synthesized scFv genes were isolated by a 1.5%-agarose gel electrophoresis. Ten micro-grams of scFv genes were obtained from individual mice. The AsiS I and Not I sites were introduced at the 5'- and 3'-ends of the scFv genes, respectively.

### (EXAMPLE 8)

### Production of polyclonal scFv antibodies with inclusion body formation.

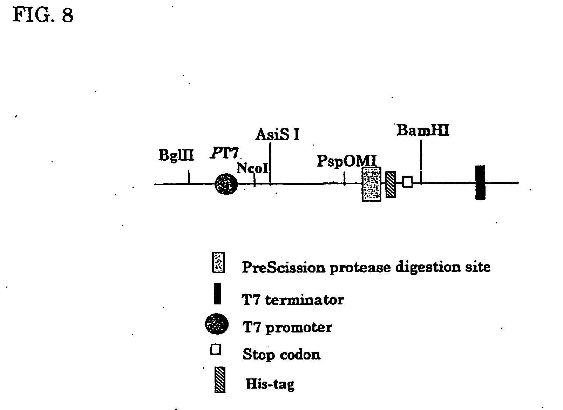

An expression vector pT3DE was constructed by insertion of a synthetic DNA fragment as shown by SEQ ID No. 82 into the Nco I-BamH I site of pET3d (Novagen Co.). The construction of pT3DE is shown in FIG. 8. The scFv genes prepared in example 7 were digested with AsiS I and Not I and the digested scFv genes (500 ng) were introduced into the AsiS I-PspOM I site of pT3DE (2 micro grams) by T4 DNA ligase. E. coli JM109 (DE3) (Clontech Co.) (300 micro liters) was transformed with the ligase-reaction-mixture containing 500 ng DNA by electroporation (2.5 kv, 25 micro F, 200 Q, 4.74 msec.). The SOC medium was added to the transformed cells and then incubated at 37 °C for 3 hours. The cell suspension (1.5 mL) was inoculated in 1.5 L of 2 X Y.T. medium containing carbenicillin (100 micro grams/mL) and culture was conducted at 35 °C for 24 hours at the rotation of 125 rpm. After the culture, cells were harvested by centrifugation. On the other hand, 100 micro liters of the 10000-fold diluted JM109 (DE3) cells transformed with ligase-reaction mixture and suspended in SOC medium was spread on LB-agar plates containing ampicillin (100 micro grams/mL) and the number of grown colonies was counted. The number of clones in the culture for polyclonal scFv antibodies production, which corresponds to the diversity of the synthesized polyclonal scFv antibodies, was estimated to be 3.5 X 10 ⁷. This efficiency was much higher than those of the PPIase fusion systems (Examples 2, 3 and 4). This indicates that diversity of synthesized polyclonal scFv antibodies in this unfused system is much larger than that of the PPIase-fusion system.

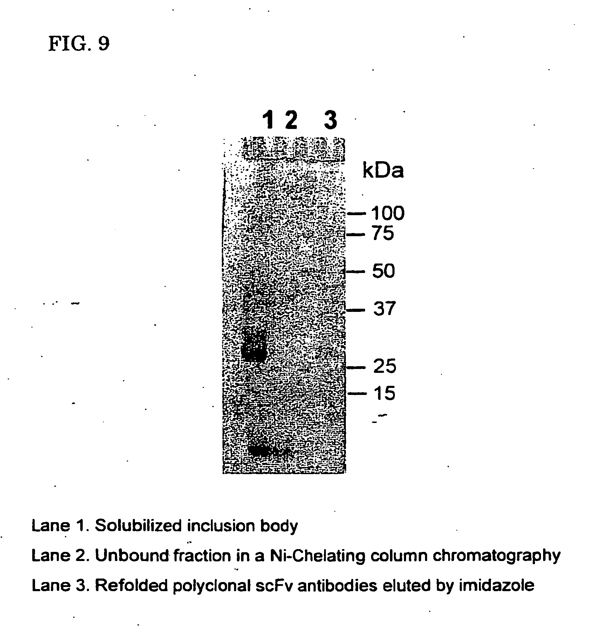

The cells were suspended in 30 mL of 50 mM Tris-HCl (pH 8.0) containing 1 mM EDTA and 200 micro liters of hen egg lysozyme (30 mg/mL). The mixture was incubated at 30 °C for 1 hour and then cells were disrupted by sonication. The supernatant was removed by centrifugation. The inclusion body was washed two times with 30 mL of 50 mM Tris-HCl (pH 8.0) containing 2% Triton X-100 and 250 mM NaCl. The washed inclusion body was solubilized in 50 mM Tris-HCl buffer containing 8M urea, 10 mM beta-mercaptoethanol (beta-ME), 0.5 M NaCl and 10 mM imidazole (buffer A, pH 8.0) at 4 °C overnight. Solubilized proteins were obtained by centrifugation. The SDS-PAGE analysis of the solubilized fraction is shown in FIG. 9. The solubilized polyclonal scFv antibodies were migrated at approximately 27-32 kDa, indicating the diversity of produced polyclonal scFv antibodies.

The supernatant was applied to a Ni-chelating column (Amersham Biosciences Co.) equilibrated with buffer A and then column was washed with 50 mM Tris-HCl buffer containing 8 M urea, 10 mM beta-ME, 0.5 M NaCl (buffer B, pH 8.0). The His-tagged polyclonal antibodies bound to Ni²⁺ were refolded by a linear gradient to 50 mM Tris-HCl buffer containing 3 M urea, 1 mM GSSG, 1 mM GSH and 0.5 M NaCl (buffer C, pH 8.0). After the refolding, the column was washed with buffer D (50 mM Tris-HCl, 3M urea, 1 mM GSSG, 1 mM GSH, 0.05 % PEG [polyethylene glycol] 4000, and 0.5 M NaCl, pH 8.0). The polyclonal scFv antibodies were further refolded by a linear gradient to buffer E (50 mM Tris-HCl, 0.5 mM GSSG, 0.05% PEG4000 and 0.5 M NaCl, pH 8.0). The refolded polyclonal scFv antibodies were eluted by buffer F (50 mM Tris-HCl, 500 mM imidazole, 0.05% PEG4000 and 0.5 M NaCl, pH 8.0). The SDS-PAGE analysis at this step is shown in FIG. 9. FIG. 9 indicates that the soluble polyclonal scFv antibodies were obtained in the absence of urea. This means that denatured polyclonal scFv antibodies were correctly refolded by the purification procedures in this example.

The eluted polyclonal scFv antibodies were dialyzed against 50 mM Tris-HCl (pH 7.0) containing 0.05 % PEG 4000, 1mM DTT and 0.15 M NaCl. To liberate the His-tag from scFv antibodies, the dialysate was digested by PreScission protease at 5 °C overnight. The digested mixture was dialyzed against 50 mM Tris-HCl (pH 7.5) containing 0.5 M NaCl, 1 mM imidazole and 0.05 % PEG4000. To remove GST (glutathione-S-transferase)-fused PreScission protease, undigested His-tagged scFv antibodies and chelating proteins from E. coli, the dialysate was applied to the tandem columns with a GST-trap (Amersham Biosciences Co.) and a His-trap column (Amersham Biosciences Co.). The unbound-fraction was collected. The collected polyclonal antibodies were dialyzed against physiological sodium chloride solution containing 2.5% glucose, 0.9% NaCl and 0.05% PEG4000. The dialysate was stored at 4 °C until the administration to the model mice of human vasculitis. The purified recombinant polyclonal scFv antibodies were obtained at the yield of 30 mg/L culture.

### (EXAMPLE 9)

### A mouse model of human vasculitis.

To make a mouse model of human vasculitis, mice were administered with CAWS (Water-soluble extracellular polysaccharide fraction obtained from the culture supernatant of Candida albicans) (N. Ohno Jpn. J. Infect. Dis. 57:S9-10, 2004).

C57BL/6N male mice (3.5 weeks) were purchased from Japan Charles River and kept in cage for 6 days before examination. The mice were grouped into seven with 10 mice in each cage and kept at 21-26 °C and 30-70% in humidity under light from 7 a.m. to 7 p.m in a pathogen free room. Solid food (Funahashi FR2: irradiated with gamma-ray (10 Gy)) was given, and sterilized tap water added with sodium hypochlorite was served for drink ad libitum. CAWS was dissolved with D-PBS (Dulbecco's phosphate buffered saline, Sigma D8537) at a concentration of 240 mg/12 mL in five tubes, respectively (total 1200 mg), and autoclaved for 20 min at 121°C. Contamination of LPS (lipopolysaccharide) in the solution was not detectable. CAWS (4mg/0.2mL/mouse) was intrapenitoneally injected into mice kept for 5 days before examination with recombinant immunoglobulin preparations.

### (EXAMPLE 10)

### Effect of the recombinant immunoglobulin preparations on the development of vasculitis in the model mice.

The recombinant CH1-CH2-CH3 (hFC123, example 5) and polyclonal scFv antibodies (poly-Fv, Example 8) were dialyzed against physiological sodium chloride solutions containing 2.5% glucose, 0.9% NaCl (saline) and containing 2.5% glucose, 0.9% NaCl and 0.05% PEG4000, respectively. After dialysis, the dialysates were treated with a 0.22-micrometer filter.

The examinations were grouped into seven groups with 10 mice in each. The seven examination groups were; 1) CAWS injection without another administration, 2) CAWS injection with saline, 3) CAWS injection with poly-Fv (170 mg protein/Kg) for 5 days, 4) CAWS injection with hFC123 (250 mg protein/Kg) for 5 days, 5) CAWS injection with poly-Fv (170 mg protein/Kg) and hFC123 (250 mg protein/Kg) for 5 days, 6) Saline injection with poly-Fv (170 mg protein/Kg) and hFC123 (250 mg protein/Kg) for 5 days, 7) CAWS injection with hIG (native immunoglobulins from human blood plasma) (500 mg protein/Kg) for 5 days. The 5-days-treatment with recombinant immunoglobulins or control samples was started 3 days after CAWS injection in vein. Recombinant immunoglobulins or control samples were also injected in vein 6-hours after injection of CAWS. Dosage of immunoglobulins was estimated in the initial body-weight of mouse. Injection in vein was carried out with a syringe pump (Harvard: Model11) at a rate of 8 min/mouse. The injection amount of recombinant immunoglobulins and hIG were adjusted to 170-500 mg protein/Kg, respectively described above. Conditions of mice were monitored in healthy for seven days after the first day of CAWS injection.

On Day-27 after finish of the CAWS injection, mice were scarified in a CO₂ gas, and blood was taken from heart with heparinezed syringe. And then, spleen and heart cut were removed and put into 10% neutralized-buffered formalin (Wako Chemicals:060-01667) for fixation. Weight of the spleen was measured and it was put into the formalin separately. Histological observations in the tissue were carried out with stained by Hematoxylin-Eosin.

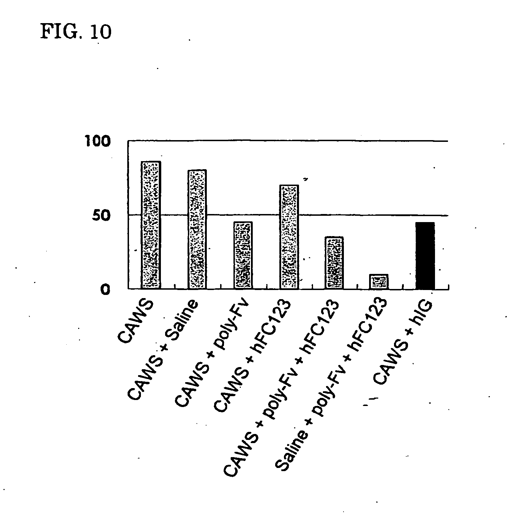

Evaluation of treatment with immunoglobulins on suppression of the development of vasculitis was carried out by histological observations. The results are summarized in FIG. 10. As results, followings were clarified; (1) Only CAWS administration induced vasculitis in about 90% mice., (2) Saline for treatment did not suppressed the development of vasculitis induced with administration of CAWS., (3) Treatment with poly-Fv injection suppressed strongly the development of vasculitis induced with administration of CAWS., (4) Treatment with hFC123 injection suppressed slightly the development of vasculitis induced with administration of CAWS., (5) Treatment with poly-Fv and hFC123 injection suppressed strongly the development of vasculitis induced with administration of CAWS., (6) Treatment with poly-Fv and hFC123 injection did not affect on any effect in mice without vasculitis, which were not administered with CAWS., (7) hIG for the control showed strongly suppression in the development of vasculitis induced with administration of CAWS. These results indicate that this invention can present safety and pharmacologically active recombinant immunoglobulin preparations.

## Claims

1. A method for producing recombinant polyclonal immunoglobulins with inclusion body formation which comprises the following steps:
(1) preparing a plurality of types of recombinant vectors into which a plurality of types of genes is introduced, respectively, by contacting a mixture of a plurality of types of genes with vectors, wherein said genes have been obtained by isolating them from cDNAs derived from tissues or cells expressing immunoglobulins, and wherein said genes encode a plurality of types of polypeptides containing a plurality of types of immunoglobulin variable regions,
and preparing a mixture of the recombinant vectors;
(2) preparing a plurality of types of transformants into which a plurality of types of recombinant vectors is introduced, respectively, by contacting said mixture of the recombinant vectors with Escherichia coli cells,
and preparing a mixture of the transformants; and
(3) culturing said mixture of the transformants,
and obtaining a mixture of polypeptides from the culture of the transformants, wherein said polypeptides contain a plurality of types of immunoglobulin variable regions, respectively, and said polypeptides when expressed form inclusion bodies,
wherein said polypeptides are scFv.

2. The method of claim 1, wherein said tissues or cells are derived from mammals.

3. The method of claim 2, wherein said mammals are mice lacking myeloperoxidase genes.

4. The method of any one of claims 1 to 3, wherein step (3) comprises obtaining inclusion bodies from the culture of the transformants, and refolding by solubilizing the inclusion bodies with denaturants so as to obtain the mixture of polypeptides containing a plurality of types of immunoglobulin variable regions.

5. The method of any one of claims 1 to 4, further comprising the step of mixing
(a) the mixture of polypeptides containing a plurality of types of immunoglobulin variable regions with
(b) polypeptides containing full-length or a part of CH1-CH2-CH3 regions.

6. The method of claim 5, wherein said polypeptides containing full-length or a part of CH1-CH2-CH3 regions according to paragraph (b) of claim 5 are obtained from a culture of transformants and said transformants contain genes encoding said polypeptides.

7. The method of claim 6, wherein said transformants are Escherichia coli.

8. The method of claim 5 or 6, wherein said polypeptides containing full-length or a part of CH1-CH2-CH3 regions according to paragraph (b) of claim 5 are expressed as a part of fusion proteins composed of folding factors and said polypeptides containing full-length or a part of CH1-CH2-CH3 regions, and isolated from the fusion proteins.

9. The method of claim 8, wherein said folding factors are peptidyl prolyl cis-trans isomerases.

10. The method of claim 9, wherein said peptidyl prolyl cis-trans isomerases are FK506 binding proteins.

11. The method of claim 10, wherein said FK506 binding proteins are derived from archaea.

12. A method for producing an immunoglobulin preparation which comprises producing recombinant polyclonal immunoglobulins by the method of any one of claims 1 to 11, and combining the recombinant polyclonal immunoglobulins with a stabilizer or an excipient.

## Patentansprüche

1. Verfahren zur Herstellung rekombinanter, polyklonaler Immunglobuline unter Bildung von Einschlusskörperchen, das die folgenden Schritte umfasst:
(1) Herstellen einer Vielzahl an Typen rekombinanter Vektoren, in die entsprechend eine Vielzahl an Gentypen eingebracht wird, indem eine Mischung einer Vielzahl an Gentypen mit Vektoren in Kontakt gebracht wird, wobei die Gene erhalten wurden, indem sie aus cDNAs isoliert wurden, die aus Immunglobulin-exprimierenden Geweben oder Zellen stammen, und wobei die Gene eine Vielzahl an Typen von Polypeptiden kodieren, welche eine Vielzahl an Typen variabler Immunglobulinregionen umfassen,
und Herstellen einer Mischung der rekombinanten Vektoren;
(2) Herstellen einer Vielzahl an Typen von Transformanten, in die entsprechend eine Vielzahl an Typen rekombinanter Vektoren eingebracht wird, indem diese Mischung rekombinanter Vektoren mit *Escherichia*-*coli*-Zellen in Kontakt gebracht wird,
und Herstellen einer Mischung der Transformanten; und
(3) Kultivieren dieser Mischung der Transformanten,
und Erhalten einer Mischung von Polypeptiden aus der Kultur der Transformanten, wobei die Polypeptide entsprechend eine Vielzahl an Typen variabler Immunglobulinregionen umfassen und die Polypeptide bei Expression Einschlusskörperchen bilden, wobei die Polypeptide scFv sind.

2. Verfahren nach Anspruch 1, wobei die Gewebe oder Zellen aus Säugetieren stammen.

3. Verfahren nach Anspruch 2, wobei die Säugetiere Mäuse sind, denen Myeloperoxidase-Gene fehlen.

4. Verfahren nach einem der Ansprüche 1 bis 3, wobei Schritt (3) umfasst:
Erhalten der Einschlusskörperchen aus der Kultur der Transformanten und
Rückfalten durch Solubilisieren der Einschlusskörperchen mittels Denaturierungsmitteln, um eine Mischung an Polypeptiden zu erhalten, die eine Vielzahl an Typen variabler Immunglobulinregionen enthalten.

5. Verfahren nach einem der Ansprüche 1 bis 4, ferner umfassend den Schritt des Mischens von
(a) der Mischung an Polypeptiden, die eine Vielzahl an Typen variabler Immunglobulinregionen enthält
mit
(b) Polypeptiden, die die gesamte Länge oder einen Teil der CH1-CH2-CH3-Regionen enthalten.

6. Verfahren nach Anspruch 5, wobei die Polypeptide gemäß Absatz (b) von Anspruch 5, die die gesamte Länge oder einen Teil der CH1-CH2-CH3-Regionen enthalten, aus einer Kultur von Transformanten erhalten werden und die Transformanten Gene enthalten, die diese Polypeptide kodieren.

7. Verfahren nach Anspruch 6, wobei die Transformanten *Escherichia coli* sind.

8. Verfahren nach Anspruch 5 oder 6, wobei die Polypeptide gemäß Absatz (b) von Anspruch 5, die die gesamte Länge oder einen Teil der CH1-CH2-CH3-Regionen enthalten, als ein Teil von Fusionsproteinen exprimiert werden, die aus Faltungsfaktoren und den Polypeptiden, die die gesamte Länge oder einen Teil der CH1-CH2-CH3-Regionen enthalten, zusammengesetzt sind, und aus den Fusionsproteinen isoliert werden.

9. Verfahren nach Anspruch 8, wobei die Faltungsfaktoren Peptidyl-Prolyl-cis/trans-Isomerasen sind.

10. Verfahren nach Anspruch 9, wobei die Peptidyl-Prolyl-cis/trans-Isomerasen FK506-Bindeproteine sind.

11. Verfahren nach Anspruch 10, wobei die FK506-Bindeproteine aus Archaeen stammen.

12. Verfahren zum Erzeugen einer Immunglobulin-Präparation, welches umfasst:
Erzeugen rekombinanter polyklonaler Immunglobuline durch das Verfahren nach irgendeinem der Ansprüche 1 bis 11, und
Kombinieren der rekombinanten polyklonalen Immunglobuline mit einem Stabilisierungsmittel oder einem Hilfsstoff.

## Revendications

1. Procédé pour la production d'immunoglobulines polyclonales recombinantes avec formation de corps d'inclusion, qui comprend les étapes suivantes:
(1) préparation d'une pluralité de types de vecteurs recombinants dans lesquels une pluralité de types de gènes est introduite, respectivement, par mise en contact d'un mélange d'une pluralité de types de gènes avec des vecteurs, tandis que lesdits gènes ont été obtenus par leur isolement à partir d'ADNc dérivés de tissus ou de cellules exprimant des immunoglobulines, et tandis que lesdits gènes codent pour une pluralité de types de polypeptides contenant une pluralité de types de régions variables d'immunoglobuline,
et préparation d'un mélange des vecteurs recombinants;
(2) préparation d'une pluralité de types de transformants dans laquelle une pluralité de types de vecteurs recombinants est introduite, respectivement, par mise en contact dudit mélange des vecteurs recombinants avec des cellules d'Escherichia coli,
et préparation d'un mélange de transformants; et
(3) culture dudit mélange des transformants,
et obtention d'un mélange de polypeptides à partir de la culture des transformants, tandis que lesdits polypeptides contiennent une pluralité de types de régions variables d'immunoglobuline, respectivement, et lesdits polypeptides lorsqu'ils sont exprimés forment des corps d'inclusion,
tandis que lesdits polypeptides sont scFv.

2. Procédé selon la revendication 1, dans lequel lesdits tissus ou cellules sont dérivés de mammifères.

3. Procédé selon la revendication 2, dans lequel lesdits mammifères sont des souris dénuées de gènes de myéloperoxydase.

4. Procédé selon l'une quelconque des revendications 1 à 3, dans lequel l'étape (3) comprend l'obtention de corps d'inclusion à partir de la culture des transformants, et le réenroulement par solubilisation des corps d'inclusion avec des dénaturants de manière à obtenir le mélange de polypeptides contenant une pluralité de types de régions variables d'immunoglobuline.

5. Procédé selon l'une quelconque des revendications 1 à 4, comprenant en outre l'étape de mélange
(a) du mélange de polypeptides contenant une pluralité de types de régions variables d'immunoglobuline
avec
(b) des polypeptides contenant la longueur totale ou une partie des régions CH1-CH2-CH3.

6. Procédé selon la revendication 5, dans lequel lesdits polypeptides contenant la longueur totale ou une partie des régions CH1-CH2-CH3 selon le paragraphe (b) de la revendication 5 sont obtenus à partir d'une culture de transformants et lesdits transformants contiennent des gènes codant pour lesdits polypeptides.

7. Procédé selon la revendication 6, dans lequel lesdits transformants sont Escherichia coli.

8. Procédé selon les revendications 5 ou 6, dans lequel lesdits polypeptides contenant la longueur totale ou une partie des régions CH1-CH2-CH3 selon le paragraphe (b) de la revendication 5 sont exprimés en tant que partie des protéines de fusion composées de facteurs d'enroulement et lesdits polypeptides contenant la longueur totale ou une partie des régions CH1-CH2-CH3, et isolés à partir des protéines de fusion.

9. Procédé selon la revendication 8, dans lequel lesdits facteurs d'enroulement sont des peptidyl prolyl cis-trans isomérases.

10. Procédé selon la revendication 9, dans lequel lesdites peptidyl prolyl cis-trans isomérases sont des protéines de liaison FK506.

11. Procédé selon la revendication 10, dans lequel lesdites protéines de liaison FK506 sont dérivées d'archaea.

12. Procédé pour la production d'une préparation d'immunoglobuline, qui comprend la production d'immunoglobulines polyclonales recombinantes par le procédé selon l'une quelconque des revendications 1 à 11, et
la combinaison des immunoglobulines polyclonales recombinantes avec un stabilisant ou un excipient.


### SEQUENCE LISTING

<110> sekisui Chemical co., Ltd
   Suzuki, Kazuo
<120> Methods for producing recombinant polyclonal immunoglobulins
<130> 471-1
<150> us 11/092988
   <151> 2005-03-30
<160> 82
<170> PatentIn version 3.1
<210> 1
   <211> 39
   <212> DNA
   <213> Artificial
<220>
   <223> Designed oligonucleotide primer for PCR
<400> 1
   gagctcggcc cagacggccg ayatccagct gactcagcc 39
<210> 2
   <211> 39
   <212> DNA
   <213> Artificial
<220>
   <223> Designed oligonucleotide primer for PCR
<400> 2
   gagctcggcc cagacggccg ayattgttct cwcccagtc 39
<210> 3
   <211> 39
   <212> DNA
   <213> Artificial
<220>
   <223> Designed oligonucleotide primer for PCR
<400> 3
   gagctcggcc cagacggccg ayattgtgmt mactcagtc 39
<210> 4
   <211> 39
   <212> DNA
   <213> Artificial
<220>
   <223> Designed oligonucleotide primer for PCR
<400> 4
   gagctcggcc cagacggccg ayattgtgyt racacagtc 39
<210> 5
   <211> 39
   <212> DNA
   <213> Artificial
<220>
   <223> Designed oligonucleotide primer for PCR
<400> 5
   gagctcggcc cagacggccg ayattgtrat gacmcagtc 39
<210> 6
   <211> 39
   <212> DNA
   <213> Artificial
<220>
   <223> Designed oligonucleotide primer for PCR
<400> 6
   gagctcggcc cagacggccg ayattmagat ramccagtc 39
<210> 7
   <211> 39
   <212> DNA
   <213> Artificial
<220>
   <223> Designed oligonucleotide primer for PCR
<400> 7
   gagctcggcc cagacggccg ayattcagat gaydcagtc 39
<210> 8
   <211> 39
   <212> DNA
   <213> Artificial
<220>
   <223> Designed oligonucleotide primer for PCR
<400> 8
   gagctcggcc cagacggccg ayatycagat gacacagac 39
<210> 9
   <211> 39
   <212> DNA
   <213> Artificial
<220>
   <223> Designed oligonucleotide primer for PCR
<400> 9
   gagctcggcc cagacggccg ayattgttct cawccagtc 39
<210> 10
   <211> 39
   <212> DNA
   <213> Artificial
<220>
   <223> Designed oligonucleotide primer for PCR
<400> 10
   gagctcggcc cagacggccg ayattgwgct sacccaatc 39
<210> 11
   <211> 39
   <212> DNA
   <213> Artificial
<220>
   <223> Designed oligonucleotide primer for PCR
<400> 11
   gagctcggcc cagacggccg ayattstrat gacccartc 39
<210> 12
   <211> 39
   <212> DNA
   <213> Artificial
<220>
   <223> Designed oligonucleotide primer for PCR
<400> 12
   gagctcggcc cagacggccg ayrttktgat gacccarac 39
<210> 13
   <211> 39
   <212> DNA
   <213> Artificial
<220>
   <223> Designed oligonucleotide primer for PCR
<400> 13
   gagctcggcc cagacggccg ayattgtgat gacbcagkc 39
<210> 14
   <211> 39
   <212> DNA
   <213> Artificial
<220>
   <223> Designed oligonucleotide primer for PCR
<400> 14
   gagctcggcc cagacggccg ayattgtgat aacycagga 39
<210> 15
   <211> 39
   <212> DNA
   <213> Artificial
<220>
   <223> Designed oligonucleotide primer for PCR
<400> 15
   gagctcggcc cagacggccg ayattgtgat gacccagwt 39
<210> 16
   <211> 39
   <212> DNA
   <213> Artificial
<220>
   <223> Designed oligonucleotide primer for PCR
<400> 16
   gagctcggcc cagacggccg ayattgtgat gacacaacc 39
<210> 17
   <211> 39
   <212> DNA
   <213> Artificial
<220>
   <223> Designed oligonucleotide primer for PCR
<400> 17
   gagctcggcc cagacggccg ayattttgct gactcagtc 39
<210> 18
   <211> 42
   <212> DNA
   <213> Artificial
<220>
   <223> Designed oligonucleotide primer for PCR
<400> 18
   gagctcggcc cagacggccg atgctgttgt gactcaggaa tc 42
<210> 19
   <211> 75
   <212> DNA
   <213> Artificial
<220>
   <223> Designed oligonucleotide primer for PCR
<400> 19
   ggaagatcta gaggaaccac ccccaccacc gcccgagcca ccgccaccag aggatttkat 60
   ttccagyttg gtccc 75
<210> 20
   <211> 75
   <212> DNA
   <213> Artificial
<220>
   <223> Designed oligonucleotide primer for PCR
<400> 20
   ggaagatcta gaggaaccac ccccaccacc gcccgagcca ccgccaccag aggattttat 60
   ttccaacttt gtccc 75
<210> 21
   <211> 75
   <212> DNA
   <213> Artificial
<220>
   <223> Designed oligonucleotide primer for PCR
<400> 21
   ggaagatcta gaggaaccac ccccaccacc gcccgagcca ccgccaccag aggatttcag 60
   ctccagcttg gtccc 75
<210> 22
   <211> 74
   <212> DNA
   <213> Artificial
<220>
   <223> Designed oligonucleotide primer for PCR
<400> 22
   ggaagatcta gaggaaccac ccccaccacc gcccgagcca ccgccaccag aggarcctag 60
   gacagtcagt ttgg 74
<210> 23
   <211> 41
   <212> DNA
   <213> Artificial
<220>
   <223> Designed oligonucleotide primer for PCR
<400> 23
   ggtggttcct ctagatcttc cgakgtrmag cttcaggagt c 41
<210> 24
   <211> 41
   <212> DNA
   <213> Artificial
<220>
   <223> Designed oligonucleotide primer for PCR
<400> 24
   ggtggttcct ctagatcttc cgaggtbcag ctbcagcagt c 41
<210> 25
   <211> 41
   <212> DNA
   <213> Artificial
<220>
   <223> Designed oligonucleotide primer for PCR
<400> 25
   ggtggttcct ctagatcttc ccaggtgcag ctgaagsavt c 41
<210> 26
   <211> 41
   <212> DNA
   <213> Artificial
<220>
   <223> Designed oligonucleotide primer for PCR
<400> 26
   ggtggttcct ctagatcttc cgaggtccar ctgcaacart c 41
<210> 27
   <211> 41
   <212> DNA
   <213> Artificial
<220>
   <223> Designed oligonucleotide primer for PCR
<400> 27
   ggtggttcct ctagatcttc ccaggtycag ctbcagcart c 41
<210> 28
   <211> 41
   <212> DNA
   <213> Artificial
<220>
   <223> Designed oligonucleotide primer for PCR
<400> 28
   ggtggttcct ctagatcttc ccaggtycar ctgcagcart c 41
<210> 29
   <211> 41
   <212> DNA
   <213> Artificial
<220>
   <223> Designed oligonucleotide primer for PCR
<400> 29
   ggtggttcct ctagatcttc ccaggtccac gtgaagcart c 41
<210> 30
   <211> 41
   <212> DNA
   <213> Artificial
<220>
   <223> Designed oligonucleotide primer for PCR
<400> 30
   ggtggttcct ctagatcttc cgaggtgaas stggtggart c 41
<210> 31
   <211> 41
   <212> DNA
   <213> Artificial
<220>
   <223> Designed oligonucleotide primer for PCR
<400> 31
   ggtggttcct ctagatcttc cgavgtgawg stggtggagt c 41
<210> 32
   <211> 41
   <212> DNA
   <213> Artificial
<220>
   <223> Designed oligonucleotide primer for PCR
<400> 32
   ggtggttcct ctagatcttc cgaggtgcag skggtggart c 41
<210> 33
   <211> 41
   <212> DNA
   <213> Artificial
<220>
   <223> Designed oligonucleotide primer for PCR
<400> 33
   ggtggttcct ctagatcttc cgakgtgcam ctggtggart c 41
<210> 34
   <211> 41
   <212> DNA
   <213> Artificial
<220>
   <223> Designed oligonucleotide primer for PCR
<400> 34
   ggtggttcct ctagatcttc cgaggtgaag ctgatggart c 41
<210> 35
   <211> 41
   <212> DNA
   <213> Artificial
<220>
   <223> Designed oligonucleotide primer for PCR
<400> 35
   ggtggttcct ctagatcttc cgaggtgcar cttgttgart c 41
<210> 36
   <211> 41
   <212> DNA
   <213> Artificial
<220>
   <223> Designed oligonucleotide primer for PCR
<400> 36
   ggtggttcct ctagatcttc cgargtraag cttctcgart c 41
<210> 37
   <211> 41
   <212> DNA
   <213> Artificial
<220>
   <223> Designed oligonucleotide primer for PCR
<400> 37
   ggtggttcct ctagatcttc cgaagtgaar sttgaggart c 41
<210> 38
   <211> 41
   <212> DNA
   <213> Artificial
<220>
   <223> Designed oligonucleotide primer for PCR
<400> 38
   ggtggttcct ctagatcttc ccaggttact ctraaasart s 41
<210> 39
   <211> 41
   <212> DNA
   <213> Artificial
<220>
   <223> Designed oligonucleotide primer for PCR
<400> 39
   ggtggttcct ctagatcttc ccaggtccaa ctvcagcarc c 41
<210> 40
   <211> 41
   <212> DNA
   <213> Artificial
<220>
   <223> Designed oligonucleotide primer for PCR
<400> 40
   ggtggttcct ctagatcttc cgatgtgaac ttggaaswrt c 41
<210> 41
   <211> 41
   <212> DNA
   <213> Artificial
<220>
   <223> Designed oligonucleotide primer for PCR
<400> 41
   ggtggttcct ctagatcttc cgaggtgaag gtcatcgart c 41
<210> 42
   <211> 45
   <212> DNA
   <213> Artificial
<220>
   <223> Designed oligonucleotide primer for PCR
<400> 42
   cctgaagcga gtgcggccgc cgacagatgg ggstgtygtt ttggc 45
<210> 43
   <211> 42
   <212> DNA
   <213> Artificial
<220>
   <223> Designed oligonucleotide primer for PCR
<400> 43
   cctgaagcga gtgcggccgc cgacagatgg ggctgttgtt gt 42
<210> 44
   <211> 45
   <212> DNA
   <213> Artificial
<220>
   <223> Designed oligonucleotide primer for PCR
<400> 44
   cctgaagcga gtgcggccgc cgacatttgg gaaggactga ctctc 45
<210> 45
   <211> 38
   <212> DNA
   <213> Artificial
<220>
   <223> Designed oligonucleotide primer for PCR
<400> 45
   gaggaggagg aggaggagga gctcggccca gacggccg 38
<210> 46
   <211> 39
   <212> DNA
   <213> Artificial
<220>
   <223> Designed oligonucleotide primer for PCR
<400> 46
   gaggaggagg aggaggagcc tgaagcgagt gcggccgcc 39
<210> 47
   <211> 27
   <212> DNA
   <213> Artificial
<220>
   <223> Designed oligonucleotide primer for PCR
<400> 47
   accatggctg aagagacaat taaaaac 27
<210> 48
   <211> 27
   <212> DNA
   <213> Artificial
<220>
   <223> Designed oligonucleotide primer for PCR
<400> 48
   tactagttgc ctccaatgat atgactt 27
<210> 49
   <211> 483
   <212> DNA
   <213> Methanosarcina acetivorans
<400> 49
   atgactgaag agacaattaa aaacccagac aaaacaatcg aaaacggaga tactatttcc 60
   gttgattatg ttggaaaact ggaagacggc actgttttcg acacatcgga aaaagaagca 120
   gcgagcgagg cagggatata caacgaaatg agggactaca aacctctgac gttcactgtg 180
   ggagcggggc agatgataaa aggctttgac gagggcgtag tcggaatgaa agtaggagag 240
   gaaaaaactc ttgaaatccc tccagaggaa gcatacggag aatacatgga agagtacgta 300
   agggaactcc cacgaaacgc agtcaatttt actcccgaaa ccgggatgca acttgctacg 360
   gaaaacgggc ttcggggaaa ggtcactgaa gtaggcgagg aaaacttcgt agtggacttc 420
   aaccatgagc ttgctggaaa gaccctgata ttcaaaataa aagtcatatc attggaggca 480
   taa 483
<210> 50
   <211> 125
   <212> DNA
   <213> Artificial
<220>
   <223> Designed oligonucleotide linker
<400> 50
   ccatggccgg cactagtctg gaagttctgt tccaggggcc gacggcccag acggccggcg 60
   gcggccgcct ggaagttctg ttccaggggc cgctgcatcg ccatcgccat cgctaatagc 120
   tcgag 125
<210> 51
   <211> 23
   <212> DNA
   <213> Artificial
<220>
   <223> Designed oligonucleotide primer for PCR
<400> 51
   tctcgagcgc ctccaccaag ggc 23
<210> 52
   <211> 21
   <212> DNA
   <213> Artificial
<220>
   <223> Designed oligonucleotide primer for PCR
<400> 52
   tcatttaccc ggagacaggg a 21
<210> 53
   <211> 1000
   <212> DNA
   <213> Homo sapiens
<400> 53
   ctcgagcgcc tccaccaagg gcccatcggt cttccccctg gcaccctcct ccaagagcac 60
   ctctgggggc acagcggccc tgggctgcct ggtcaaggac tacttccccg aaccggtgac 120
   ggtgtcgtgg aactcaggcg ccctgaccag cggcgtgcac accttcccgg ctgtcctaca 180
   gtcctcagga ctctactccc tcagcagcgt ggtgaccgtg ccctccagca gcttgggcac 240
   ccagacctac atctgcaacg tgaatcacaa gcccagcaac accaaggtgg acaagaaagt 300
   tgagcccaaa tcttgtgaca aaactcacac atgcccaccg tgcccagcac ctgaactcct 360
   ggggggaccg tcagtcttcc tcttcccccc aaaacccaag gacaccctca tgatctcccg 420
   gacccctgag gtcacatgcg tggtggtgga cgtgagccac gaagaccctg aggtcaagtt 480
   caactggtac gtggacggcg tggaggtgca taatgccaag acaaagccgc gggaggagca 540
   gtacaacagc acgtaccgtg tggtcagcgt cctcaccgtc ctgcaccagg actggctgaa 600
   tggcaaggag tacaagtgca aggtctccaa caaagccctc ccagccccca tcgagaaaac 660
   catctccaaa gccaaagggc agccccgaga accacaggtg tacaccctgc ccccatcccg 720
   ggatgagctg accaagaacc aggtcagcct gacctgcctg gtcaaaggct tctatcccag 780
   cgacatcgcc gtggagtggg agagcaatgg gcagccggag aacaactaca agaccacgcc 840
   tcccgtgctg gactccgacg gctccttctt cctctacagc aagctcaccg tggacaagag 900
   caggtggcag caggggaacg tcttctcatg ctccgtgatg catgagggtc tgcacaacca 960
   ctacacgcag aagagcctct ccctgtctcc gggtaaatga 1000
<210> 54
   <211> 32
   <212> DNA
   <213> Artificial
<220>
   <223> Designed oligonucleotide linker
<400> 54
   gcggccgcgg atccgcacta gaattcctcg ag 32
<210> 55
   <211> 20
   <212> DNA
   <213> Artificial
<220>
   <223> Designed oligonucleotide primer for PCR
<400> 55
   aggctgccca ccgtgcccag 20
<210> 56
   <211> 23
   <212> DNA
   <213> Artificial
<220>
   <223> Designed oligonucleotide primer for PCR
<400> 56
   accgtcattt acccggagac agg 23
<210> 57
   <211> 669
   <212> DNA
   <213> Homo sapiens
<400> 57
   tgcccaccgt gcccagcacc tgaactcctg gggggaccgt cagtcttcct cttcccccca 60
   aaacccaagg acaccctcat gatctcccgg acccctgagg tcacatgcgt ggtggtggac 120
   gtgagccacg aagaccctga ggtcaagttc aactggtacg tggacggcgt ggaggtgcat 180
   aatgccaaga caaagccgcg ggaggagcag tacaacagca cgtaccgtgt ggtcagcgtc 240
   ctcaccgtcc tgcaccagga ctggctgaat ggcaaggagt acaagtgcaa ggtctccaac 300
   aaagccctcc cagcccccat cgagaaaacc atctccaaag ccaaagggca gccccgagaa 360
   ccacaggtgt acaccctgcc cccatcccgg gatgagctga ccaagaacca ggtcagcctg 420
   acctgcctgg tcaaaggctt ctatcccagc gacatcgccg tggagtggga gagcaatggg 480
   cagccggaga acaactacaa gaccacgcct cccgtgctgg actccgacgg ctccttcttc 540
   ctctacagca agctcaccgt ggacaagagc aggtggcagc aggggaacgt cttctcatgc 600
   tccgtgatgc atgaggctct gcacaaccac tacacgcaga agagcctctc cctgtctccg 660
   ggtaaatga 669
<210> 58
   <211> 39
   <212> DNA
   <213> Artificial
<220>
   <223> Designed oligonucleotide linker
<400> 58
   actagtctgg aagttctgtt ccaggggccg acgggatcc 39
<210> 59
   <211> 27
   <212> DNA
   <213> Artificial
<220>
   <223> Designed oligonucleotide primer for CDNA synthesis
<400> 59
   gctggagggk acagtcactg agctgct 27
<210> 60
   <211> 29
   <212> DNA
   <213> Artificial
<220>
   <223> Designed oligonucleotide primer for cDNA synthesis
<400> 60
   gatgacttca gtgttgttct ggtagttcc 29
<210> 61
   <211> 30
   <212> DNA
   <213> Artificial
<220>
   <223> Designed oligonucleotide primer for cDNA synthesis
<400> 61
   ttccacttga cattgatgtc tttggggtag 30
<210> 62
   <211> 40
   <212> DNA
   <213> Artificial
<220>
   <223> Designed oligonucleotide primer for PCR
<400> 62
   ctcgagccat gggcgatcgc gayatccagc tgactcagcc 40
<210> 63
   <211> 40
   <212> DNA
   <213> Artificial
<220>
   <223> Designed oligonucleotide primer for PCR
<400> 63
   ctcgagccat gggcgatcgc gayattgttc tcwcccagtc 40
<210> 64
   <211> 40
   <212> DNA
   <213> Artificial
<220>
   <223> Designed oligonucleotide primer for PCR
<400> 64
   ctcgagccat gggcgatcgc gayattgtgm tmactcagtc 40
<210> 65
   <211> 40
   <212> DNA
   <213> Artificial
<220>
   <223> Designed oligonucleotide primer for PCR
<400> 65
   ctcgagccat gggcgatcgc gayattgtgy tracacagtc 40
<210> 66
   <211> 40
   <212> DNA
   <213> Artificial
<220>
   <223> Designed oligonucleotide primer for PCR
<400> 66
   ctcgagccat gggcgatcgc gayattgtra tgacmcagtc 40
<210> 67
   <211> 40
   <212> DNA
<213> Artificial
<220>
   <223> Designed oligonucleotide primer for PCR
<400> 67
   ctcgagccat gggcgatcgc gayattmaga tramccagtc 40
<210> 68
   <211> 40
   <212> DNA
   <213> Artificial
<220>
   <223> Designed oligonucleotide primer for PCR
<400> 68
   ctcgagccat gggcgatcgc gayattcaga tgaydcagtc 40
<210> 69
   <211> 40
   <212> DNA
   <213> Artificial
<220>
   <223> Designed oligonucleotide primer for PCR
<400> 69
   ctcgagccat gggcgatcgc gayatycaga tgacacagac 40
<210> 70
   <211> 40
   <212> DNA
   <213> Artificial
<220>
   <223> Designed oligonucleotide primer for PCR
<400> 70
   ctcgagccat gggcgatcgc gayattgttc tcawccagtc 40
<210> 71
   <211> 40
   <212> DNA
   <213> Artificial
<220>
   <223> Designed oligonucleotide primer for PCR
<400> 71
   ctcgagccat gggcgatcgc gayattgwgc tsacccaatc 40
<210> 72
   <211> 40
   <212> DNA
   <213> Artificial
<220>
   <223> Designed oligonucleotide primer for PCR
<400> 72
   ctcgagccat gggcgatcgc gayattstra tgacccartc 40
<210> 73
   <211> 40
   <212> DNA
   <213> Artificial
<220>
   <223> Designed oligonucleotide primer for PCR
<400> 73
   ctcgagccat gggcgatcgc gayrttktga tgacccarac 40
<210> 74
   <211> 40
   <212> DNA
   <213> Artificial
<220>
   <223> Designed oligonucleotide primer for PCR
<400> 74
   ctcgagccat gggcgatcgc gayattgtga tgacbcagkc 40
<210> 75
   <211> 40
   <212> DNA
   <213> Artificial
<220>
   <223> Designed oligonucleotide primer for PCR
<400> 75
   ctcgagccat gggcgatcgc gayattgtga taacycagga 40
<210> 76
   <211> 40
   <212> DNA
   <213> Artificial
<220>
   <223> Designed oligonucleotide primer for PCR
<400> 76
   ctcgagccat gggcgatcgc gayattgtga tgacccagwt 40
<210> 77
   <211> 40
   <212> DNA
   <213> Artificial
<220>
   <223> Designed oligonucleotide primer for PCR
<400> 77
   ctcgagccat gggcgatcgc gayattgtga tgacacaacc 40
<210> 78
   <211> 40
   <212> DNA
   <213> Artificial
<220>
   <223> Designed oligonucleotide primer for PCR
<400> 78
   ctcgagccat gggcgatcgc gayattttgc tgactcagtc 40
<210> 79
   <211> 43
   <212> DNA
   <213> Artificial
<220>
   <223> Designed oligonucleotide primer for PCR
<400> 79
   ctcgagccat gggcgatcgc gatgctgttg tgactcagga atc 43
<210> 80
   <211> 39
   <212> DNA
   <213> Artificial
<220>
   <223> Designed oligonucleotide primer for PCR
<400> 80
   gaggaggagg aggaggagct cgagccatgg gcgatcgcg 39
<210> 81
   <211> 38
   <212> DNA
   <213> Artificial
<220>
   <223> Designed oligonucleotide primer for PCR
<400> 81
   gaggaggagg aggaggagcc tgaagcgagt gcggccgc 38
<210> 82
   <211> 89
   <212> DNA
   <213> Artificial
<220>
   <223> Designed oligonucleotide linker
<400> 82
   catgggcgat cgccatcgag cggccgccac gggccctctg gaagttctgt tccaggggcc    60
   gctgcatcgc catcgccatc gctaatagg                                      89